(19) Europäisches Patentamt
European Patent Office
Office européen des brevets

(11) **EP 4 765 131 A1**

(12) **EUROPEAN PATENT APPLICATION**
published in accordance with Art. 153(4) EPC

(43) Date of publication:
**24.06.2026 Bulletin 2026/26**

(21) Application number: **25841575.1**

(22) Date of filing: **17.07.2025**

(51) International Patent Classification (IPC):
*G16B 40/00* (2019.01)    *G16B 15/20* (2019.01)
*G16B 20/00* (2019.01)    *G06N 3/08* (2023.01)
*C12Q 1/00* (2006.01)    *G16B 40/20* (2019.01)
*G16C 20/70* (2019.01)    *G16B 30/10* (2019.01)
*G16C 20/30* (2019.01)    *G16C 20/40* (2019.01)

(52) Cooperative Patent Classification (CPC):
C12Q 1/00; G06N 3/0455; G06N 3/0475;
G06N 3/08; G06N 3/0895; G06N 3/0985;
G16B 15/20; G16B 15/30; G16B 20/00;
G16B 30/10; G16B 40/00; G16B 40/20;
G16C 20/10; G16C 20/30; G16C 20/40;    (Cont.)

(86) International application number:
**PCT/KR2025/010584**

(87) International publication number:
**WO 2026/019284 (22.01.2026 Gazette 2026/04)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **18.07.2024  KR 20240095089
30.07.2024  KR 20240100992**

(71) Applicant: **LG Chem, Ltd.
Seoul 07336 (KR)**

(72) Inventors:
• **HEO, Sungwoon
Daejeon 34122 (KR)**
• **YEU, Yunku
Daejeon 34122 (KR)**
• **HEO, Hyunyoung
Daejeon 34122 (KR)**
• **LEE, Kyu Hwang
Daejeon 34122 (KR)**
• **JO, Seongin
Daejeon 34122 (KR)**

(74) Representative: **Goddar, Heinz J.
Boehmert & Boehmert
Anwaltspartnerschaft mbB
Pettenkoferstrasse 22
80336 München (DE)**

(54) **METHOD AND DEVICE FOR DISCOVERING ENZYME**

(57)    A method for searching an enzyme performed by a computing device including a processor and a memory includes: loading, by the processor, a first encoder in which learning is performed based on a functional feature of the enzyme into the memory; loading, by the processor, a second encoder in which learning is performed based on a structural feature of a compound into the memory; constructing, by the processor, a combination network that combines the first encoder and the second encoder through transfer learning; inputting, by the processor, an amino acid sequence of a candidate enzyme and graph data of a target compound in the first encoder and the second encoder and calculating, by the processor, a reaction probability in which the candidate enzyme and the target compound will react through the combination network to store the calculated reaction probability in the memory; and selecting, by the processor, a final candidate enzyme that performs a reaction on a biosynthetic pathway for producing the target compound among candidate enzymes based on the calculated reaction probability and storing, by the processor, biosynthetic pathway data that optimizes the biosynthetic pathway using the final candidate enzyme in the memory.

EP 4 765 131 A1

【Figure 1】

| First encoder providing module (110) | Second encoder providing module (120) |
|---|---|
| Combination network construction module (130) | Reaction probability prediction module (140) |
| Learning module (150) | Final candidate enzyme selection module (160) |

(52) Cooperative Patent Classification (CPC): (Cont.)
    **G16C 20/70**

# EP 4 765 131 A1

**Description**

**[Technical Field]**

Cross-reference to related applications

This application claims priority to and the benefit of Korean Patent Application No. 10-2024-0095089 filed with the Korean Intellectual Property Office on July 18, 2024, and Korean Patent Application No. 10-2024-0100992 filed with the Korean Intellectual Property Office on July 30, 2024, the entire contents of which are incorporated herein by reference.

The present disclosure relates to a method and a device for searching an enzyme, and more particularly, to a method and a device for searching an enzyme that search a candidate enzyme and search an enzyme through selection of a final candidate enzyme.

**[Background Art]**

As interest in sustainability increases worldwide, research on ways mitigating climate change by reducing a greenhouse gas is active. A bio-based material may play an important role in reducing greenhouse gas emission by replacing a conventional fossil fuel-based material. Polylactic acid (PLA) that is an example of the bio-based material may be a type of bioplastic, and may be produced by converting a carbohydrate such as glucose or starch into lactic acid through microbial fermentation from a renewable plant resource such as corn, sugarcane, or potato and synthesizing and polymerizing lactide. Bio-based 1,4-butanediol (Bio-BDO) that is an another example of the bio-based material may be 1,4-butanediol (1,4-BDO) produced from renewable biomass, and may be produced through a microbial fermentation process without relying on a fossil fuel. Bio-based 1,4-butanediol (Bio-BDO) may be applied in various applications such as being converted to tetrahydrofuran (THF) through a dehydration reaction.

An industrial strain may be a microbial strain selected and optimized for mass production of an industrially useful product or material, and may be essential for production of the bio-based material, and may play a key role in converting biomass into various high value-added products. A traditional method for developing the industrial strain includes mutation induction, gene recombination, metabolic engineering, and adaptive evolution. However, efforts are being made to maximize a research efficiency through data-based prediction and optimization using a rapidly developing artificial intelligence technology.

**[Disclosure]**

**[Technical Problem]**

A problem to be solved by the present disclosure is intended to provide a method and a device for searching an enzyme that may search an enzyme capable of reacting with a designated target compound using an artificial intelligence technology based on a functional feature of the enzyme and a structural feature of a compound.

Another problem to be solved by the present disclosure is intended to provide a method and a device for searching an enzyme that may provide prediction of a function of the enzyme capable of improving prediction accuracy for functional annotation having a hierarchical structure and solving a data imbalance problem.

**[Technical Solution]**

A method for searching an enzyme according to an embodiment of the present disclosure includes: loading, by the processor, a first encoder in which learning is performed based on a functional feature of the enzyme into the memory; loading, by the processor, a second encoder in which learning is performed based on a structural feature of a compound into the memory; constructing, by the processor, a combination network that combines the first encoder and the second encoder through transfer learning; inputting, by the processor, an amino acid sequence of a candidate enzyme and graph data of a target compound in the first encoder and the second encoder and calculating, by the processor, a reaction probability in which the candidate enzyme and the target compound will react through the combination network to store the calculated reaction probability in the memory; and selecting, by the processor, a final candidate enzyme that performs a reaction on a biosynthetic pathway for producing the target compound among candidate enzymes based on the calculated reaction probability and storing, by the processor, biosynthetic pathway data that optimizes the biosynthetic pathway using the final candidate enzyme in the memory.

The constructing of the combination network may include combining, by the processor, a first feature extracted from the first encoder and a second feature extracted from the second encoder into one vector through a fully connected layer, and the calculating the reaction probability to store the calculated reaction probability in the memory may include nonlinearly transforming, by the processor, the vector by applying a nonlinear activation function to the fully connected layer to calculate the reaction probability.

The first encoder may learn a reaction feature of the enzyme through a task of predicting an enzyme commission (EC) number from an amino acid sequence of the enzyme, and may perform multi-class prediction for a multi-level of the enzyme commission number using contrastive learning.

The first encoder may be learned to generate two positive samples through data augmentation, maximize similarity between the positive samples through a contrastive loss function, and minimize similarity between negative samples.

The second encoder may map a graph of the target compound to a continuous number-based latent space based on a generative model, and may extract a feature for a junction in a unit of a junction tree greater than a unit of an atom.

The second encoder may further include a network that predicts energy of a reactant from a mapping value of the latent space.

The first encoder may include an enzyme function prediction model that predicts enzyme function through prediction of functional annotation of a protein; and the method may further include: acquiring, by the processor, learning data related to the functional feature of the enzyme; respectively introducing, by the processor, a plurality of basic losses for a plurality of activation layers corresponding to a plurality of levels; applying, by the processor, data augmentation to the enzyme function prediction model and introducing, by the processor, an additional loss; defining, by the processor, a total loss function based on the plurality of basic losses and the additional loss; and learning, by the processor, the enzyme function prediction model based on the learning data and the total loss function.

The plurality of basic losses may include a first basic loss to an Mth basic loss, and M may be an integer greater than or equal to 2; and the defining of the total loss function may include defining the total loss function (I) according to Equation 1 below:

(Equation 1)

$$l = \sum_{k=1}^{M} w_{cce,k} \cdot l_{cce,k} + w_{sim} \cdot l_{sim}.$$

In Equation 1 above, $l_{cce,k}$ may be a kth basic loss among the plurality of basic losses (where k is an integer greater than or equal to 1 and less than or equal to M), $l_{sim}$ may be the additional loss, $w_{cce,k}$ may be a predetermined weight for the kth basic loss, and $w_{sim}$ may be a predetermined weight for the additional loss.

The additional loss may be defined according to Equation 2 below:

(Equation 2)

$$l_{sim} = -\sum_{i,j} \log \frac{\exp\left(\frac{sim(z_i, z_j)}{\tau}\right)}{\sum_{k=1}^{2N} 1_{[k \neq i]} \exp\left(\frac{sim(z_i, z_j)}{\tau}\right)}.$$

In Equation 2 above, $z_i$ and $z_j$ may be an embedding vector of a positive pair, $sim(z_i, z_j)$ may be cosine similarity of $z_i$ and $z_j$, $\tau$ may be a temperature parameter, N may be a batch size, and $1_{[k \neq i]}$ may represent 1 when $k \neq i$.

The additional loss may be defined according to Equation 3 below:

(Equation 3)

$$l_{sim} = -\sum_{i,j} \log \frac{\mathbb{N}_{i,k} \exp\left(\frac{sim(z_i, z_j)}{\tau}\right)}{\sum_{k=1}^{2N} \mathbb{N}_{i,k} \exp\left(\frac{sim(z_i, z_j)}{\tau}\right)}.$$

In Equation 3 above, $\mathbb{N}_{i,k}$ may be a multi-level term, $z_i$ and $z_j$ may be an embedding vector of a positive pair,

$sim(z_i, z_j)$ may be cosine similarity of $z_i$ and $z_j$, $\tau$ may be a temperature parameter, N may be a batch size, and $1_{[k \neq i]}$ may represent 1 when $k \neq i$.

A device for searching an enzyme according to an embodiment of the present disclosure includes: one or more non-transitory computer-readable media that include an instruction; and one or more processors that perform an operation by executing the instruction. The operation includes: loading a first encoder in which learning is performed based on a functional feature of the enzyme into the media; loading a second encoder in which learning is performed based on a structural feature of a compound into the media; constructing a combination network that combines the first encoder and the second encoder through transfer learning; inputting an amino acid sequence of a candidate enzyme and graph data of a target compound in the first encoder and the second encoder and calculating a reaction probability in which the candidate enzyme and the target compound will react through the combination network to store the calculated reaction probability in the media; and selecting a final candidate enzyme that performs a reaction on a biosynthetic pathway for producing the target compound among candidate enzymes based on the calculated reaction probability and storing biosynthetic pathway data that optimizes the biosynthetic pathway using the final candidate enzyme in the media.

The constructing of the combination network may include combining a first feature extracted from the first encoder and a second feature extracted from the second encoder into one vector through a fully connected layer, and the calculating of the reaction probability may include nonlinearly transforming the vector by applying a nonlinear activation function to the fully connected layer to predict the reaction probability.

The first encoder may learn a reaction feature of the enzyme through a task of predicting an enzyme commission (EC) number from an amino acid sequence of the enzyme, and may perform multi-class prediction for a multi-level of the enzyme commission number using contrastive learning.

The first encoder may be learned to generate two positive samples through data augmentation, maximize similarity between the positive samples through a contrastive loss function, and minimize similarity between negative samples.

The second encoder may map a graph of the target compound to a continuous number-based latent space based on a generative model, and may extract a feature for a junction in a unit of a junction tree greater than a unit of an atom.

The second encoder may further include a network that predicts energy of a reactant from a mapping value of the latent space.

The first encoder may include an enzyme function prediction model that predicts enzyme function through prediction of functional annotation of a protein; and the operation may further include: acquiring learning data related to the functional feature of the enzyme; respectively introducing a plurality of basic losses for a plurality of activation layers corresponding to a plurality of levels; applying data augmentation to the enzyme function prediction model and introducing an additional loss; defining a total loss function based on the plurality of basic losses and the additional loss; and learning the enzyme function prediction model based on the learning data and the total loss function.

The plurality of basic losses may include a first basic loss to an Mth basic loss, and M may be an integer greater than or equal to 2; and the defining of the total loss function may include defining the total loss function (I) according to Equation 1 below:

(Equation 1)

$$l = \sum_{k=1}^{M} w_{cce,k} \cdot l_{cce,k} + w_{sim} \cdot l_{sim}.$$

In Equation 1 above, $l_{cce,k}$ may be a kth basic loss among the plurality of basic losses (where k is an integer greater than or equal to 1 and less than or equal to M), $l_{sim}$ may be the additional loss, $w_{cce,k}$ may be a predetermined weight for the kth basic loss, and $w_{sim}$ may be a predetermined weight for the additional loss.

The additional loss may be defined according to Equation 2 below:

(Equation 2)

$$l_{sim} = -\sum_{i,j} \log \frac{\exp\left(\frac{sim(z_i, z_j)}{\tau}\right)}{\sum_{k=1}^{2N} 1_{[k \neq i]} \exp\left(\frac{sim(z_i, z_j)}{\tau}\right)}.$$

In Equation 2 above, $z_i$ and $z_j$ may be an embedding vector of a positive pair, $sim(z_i, z_j)$ may be cosine similarity of $z_i$ and $z_j$, $\tau$ may be a temperature parameter, N may be a batch size, and $1_{[k \neq i]}$ may represent 1 when $k \neq i$.

The additional loss may be defined according to Equation 3 below:

(Equation 3)

$$l_{sim} = -\sum_{i,j} \log \frac{\mathbb{N}_{i,k} \exp\left(\frac{sim(z_i, z_j)}{\tau}\right)}{\sum_{k=1}^{2N} \mathbb{N}_{i,k} \exp\left(\frac{sim(z_i, z_j)}{\tau}\right)}.$$

In Equation 3 above, $\mathbb{N}_{i,k}$ may be a multi-level term, $z_i$ and $z_j$ may be an embedding vector of a positive pair, $sim(z_i, z_j)$ may be cosine similarity of $z_i$ and $z_j$, $\tau$ may be a temperature parameter, N may be a batch size, and $1_{[k \neq i]}$ may represent 1 when $k \neq i$.

[Advantageous Effects]

According to embodiments, an artificial intelligence technology based on a functional feature of an enzyme and a structural feature of a compound may be used to search the enzyme capable of reacting with a designated target compound. According to the embodiments, through an artificial intelligence model that learns a function of the enzyme and transfer learning, it is possible to predict a possibility of a reaction with the target compound without being limited to predicting the function of the enzyme. Accordingly, according to the embodiments, it is possible to discover a promising enzyme that may perform a specific reaction well and accelerate development of a microbial strain that produces an eco-friendly bio-based material.

Additionally, the embodiments may provide prediction of the function of the enzyme, and in order to perform learning of an enzyme function prediction model, the embodiments may define a total loss function by introducing a plurality of basic losses corresponding to a hierarchical structure and an additional loss related to data augmentation. Accordingly, the embodiments may reflect a hierarchical structure of functional annotation to position features of enzymes with the same class at each level sufficiently close to each other, and may improve prediction accuracy for the functional annotation having the hierarchical structure and may solve a data imbalance problem.

[Description of the Drawings]

FIG. 1 is a view for describing a device for searching an enzyme according to an embodiment.
FIG. 2 is a view for describing a device for searching an enzyme according to an embodiment.
FIG. 3 is a view for describing a generative model related to a second encoder according to an embodiment.
FIG. 4 is a view for describing a second encoder according to an embodiment.
FIG. 5 is a view for describing a method for searching an enzyme according to an embodiment.
FIG. 6 is a view for describing a device for predicting a function of the enzyme according to an embodiment.
FIG. 7 is a view for describing a device for predicting a function of the enzyme according to an embodiment.
FIG. 8 is a view for describing a device for predicting a function of the enzyme according to an embodiment.
FIG. 9 is a view for describing a method for searching the enzyme based on prediction of the function of the enzyme according to an embodiment.
FIG. 10 is a view for describing a computing device according to an embodiment.

[Mode for Invention]

An embodiment of the present disclosure will be described in detail hereinafter with reference to the accompanying drawings so that those skilled in the art could easily implement the embodiment. The present disclosure may be modified in various ways, all without departing from the spirit or scope of the present disclosure. In order to clearly describe the present disclosure, parts or portions that are irrelevant to the description are omitted in the drawings, and similar constituent elements throughout the specification are denoted by similar reference numerals.

Unless explicitly stated to the contrary, the word "comprise" and variations such as "comprises" and "comprising" in the specification and the claims should be understood to imply the inclusion of stated elements but not the exclusion of any other elements. Terms including ordinal numbers such as "first" and "second" may be used to describe various constituent elements, but the constituent elements are not limited by the terms. The terms may be used for a purpose of distinguishing one constituent element from other constituent elements.

Terms such as a "unit", "er/or", a "module", and the like described in the specification may mean a unit for processing at least one function or operation, and may be implemented by hardware, software, or a combination of hardware and software. At least some components or functions of a method and a device for searching an enzyme according to embodiments described below may be implemented as a program or software, and the program or the software may be stored in a computer-readable recording medium or storage medium.

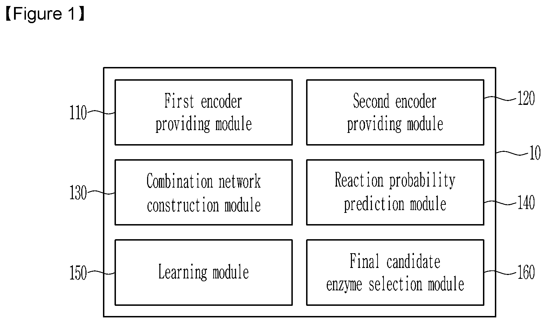

FIG. 1 is a view for describing the device for searching the enzyme according to the embodiment.

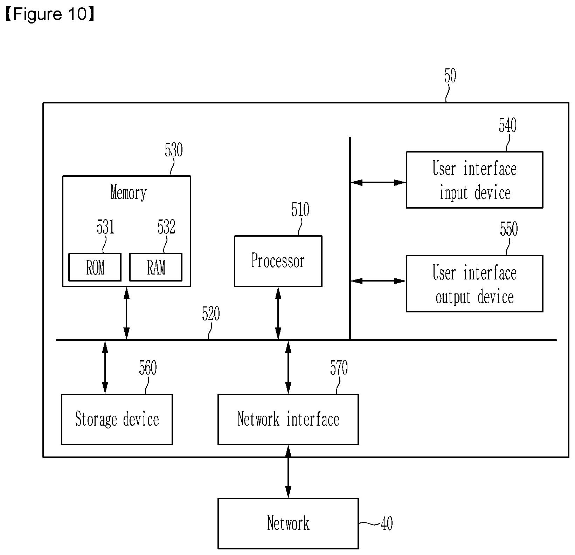

Referring to FIG. 1, a device for searching an enzyme 10 according to an embodiment may be implemented as a computing device including a processor and a memory. For example, the device for searching the enzyme 10 may be implemented as a computing device 50 described below with reference to FIG. 10. In this case, the processor may correspond to a processor 510 of the computing device 50, and the memory may correspond to a memory 520 of the computing device 50. Alternatively, in some embodiments, the device for searching the enzyme 10 may include one or more non-transitory computer-readable media including an instruction and one or more processors that execute the instruction to perform an operation. Here, the operation may include an element, a function, a step, or the like described in the present specification regarding the method and the device for searching the enzyme according to the embodiments. In the present specification, a term such as "module" is used to logically distinguish operations performed by the method and the device for searching the enzyme according to the embodiments.

Development of a strain producing the target compound may be accomplished through several processes including selection of the strain, design of a metabolic pathway, selection of a highly resistant strain, optimization of a metabolic flow, fermentation, and separation/purification/scale-up. The selection of the strain may refer to selection of a microbial strain capable of producing the target compound. The selection of the strain may search the strain producing the target compound in various environments in nature, may select a strain that is advantageous for producing the target compound among strains that have already been studied, or may use a strain held by a microbial strain bank. The design of the metabolic pathway may be design and optimization of the metabolic pathway of the strain to efficiently produce the target compound, and may include steps such as search of a biosynthetic pathway of the target compound, selection of a candidate enzyme, and development of a high-performance enzyme through mutation. The selection of the highly resistant strain may be selection of a strain that may survive even at a high concentration of the target compound, and the design of the metabolic pathway may be maximizing productivity by optimizing a production flow of the target compound within the metabolic pathway. The fermentation may be producing the target compound by culturing the optimized strain in a large quantity, and the separation/purification/scale-up may be producing the target compound on an industrial scale by efficiently separating and purifying the target compound after the fermentation.

A process of converting glucose into ethanol that is an example of a metabolic pathway may be performed through glycolysis and ethanol fermentation. For example, the glucose may be decomposed into pyruvate through the glycolysis. Here, the glycolysis may be a series of enzymatic catalytic reactions that decompose one glucose molecule into two pyruvate molecules. The pyruvate may be decarboxylated by pyruvate decarboxylase to be converted into acetaldehyde. Subsequently, the acetaldehyde may be reduced to ethanol by alcohol dehydrogenase. That is, the ethanol that is a final product may be generated as a result of alcoholic fermentation.

With development of genomics and proteomics technologies, numerous new protein sequences are being registered in databases. However, only a few of the registered proteins may directly and experimentally confirm their function. Therefore, functional annotation of most proteins may be achieved through computer-based prediction. The functional annotation of the protein may help accelerate development of a new drug and a novel bio-based material.

The functional annotation of the protein may be assignment of biological meaning to a sequence or a structure of the protein to describe and understand a function of the protein. For example, the functional annotation may have categories such as a molecular functional aspect including a specific activity or function performed by the protein, a biological process aspect including a life processe or a pathway in which the protein is involved, and a cellular compositional aspect including an intracellular position where the protein acts. A representative example of the functional annotation may be an enzyme commission (EC) number.

The EC number may be a numbering system used internationally to systematically classify enzymes. The EC number system may be used to classify and name the enzymes according to their chemical reaction mechanism. The EC number may include four numbers, each number may be separated by a dot, and each number may represent a specific attribute and a reaction type of the enzyme. For example, a first number may be a main layer item and may indicate a general type of reaction catalyzed by the enzyme, and a second number may be a sub-layer item and may indicate a more specific type of reaction. A third number may be a sub-sub-layer item and may indicate a more detailed type of reaction within the sub-layer item, and a fourth number may be a naming layer item and may correspond to a unique number that distinguishes a particular enzyme.

Hereinafter, for clarity and convenience of description, embodiments will be mainly described using the EC number as an example, but a range of the functional annotation to which an idea of the present disclosure is applied is not limited to the EC number, and may extend to any functional annotation having a hierarchical structure.

In the metabolic pathway where glucose is converted to ethanol, the EC number of pyruvate decarboxylase may be EC 4.1.1.1, and gene ontology representing pyruvate decarboxylase activity may be GO:0004736. EC 4.1.1.1 may provide a specific classification of pyruvate decarboxylase as follows.

EC 4: Lyases - an enzyme that breaks down a compound by generating or removing a double bond of a substrate
EC 4.1: Carbon-Carbon Lyases - an enzyme that cuts a carbon-carbon bond
EC 4.1.1: Carboxy-Lyases - an enzyme that catalyzes a decarboxylation reaction of carboxylic acid
EC 4.1.1.1: Pyruvate decarboxylase
GO:0004736 may be GO that represents the pyruvate decarboxylase activity to describe a molecular function of the enzyme, and may define activity of the enzyme that catalyzes a reaction that converts pyruvate into acetaldehyde and carbon dioxide.
The EC number of alcohol dehydrogenase may be EC 1.1.1.1, and GO indicating alcohol dehydrogenase activity may be GO:0004022. EC 1.1.1.1 may provide a specific classification of alcohol dehydrogenase as follows.
EC 1: Oxidoreductases - an enzyme that catalyzes an oxidation-reduction reaction
EC 1.1: Oxidoreductases whose functional group is alcohol or polyol
EC 1.1.1: Oxidoreductases that use $NAD^+$ or $NADP^+$ as a receptor
EC 1.1.1.1: Alcohol dehydrogenase
GO:0004022 is GO that represents the alcohol dehydrogenase activity to describe a molecular function of the enzyme, and may define enzyme activity that oxidizes alcohol to aldehyde or ketone using $NAD^+$ as an receptor.

After the biosynthetic pathway of the target compound is searched, a candidate enzyme capable of performing a reaction corresponding to each step should be found. As exemplified above, a biochemical reaction by the enzyme may be classified by the EC number, the GO, or the like so that an enzyme candidate group is selected using the classification. That is, a previously unknown biochemical reaction may search the EC number or the GO that performs the most similar reaction based on chemical similarity between the a reactant and a product. However, a method of compressing the candidate enzyme through the EC number or the GO may have a limitation in that it requires numerous trials and errors to find a highly productive enzyme.

To overcome the limitation and reduce trial and error in search for the highly productive enzyme, the device for searching the enzyme 10 may provide an artificial intelligence model that inputs a graph of the compound along with an amino acid sequence of the enzyme and outputs a probability in which the enzyme and the compound will react. The artificial intelligence model may be different from most conventional enzyme function prediction models that input only an amino acid sequence of the enzyme and output a probability distribution of a classified enzyme function. For example, the device for searching the enzyme 10 may include a first encoder providing module 110, a second encoder providing module 120, a combination network construction module 130, a reaction probability prediction module 140, a learning module 150, and a final candidate enzyme selection module 160 to search for the enzyme capable of reacting with the compound.

The first encoder providing module 110 may provide a first encoder in which learning is performed based on a functional feature of the enzyme, and the second encoder providing module 120 may provide a second encoder in which learning is performed based on a structural feature of the compound. For example, the first encoder providing module 110 may load the first encoder into a memory, and the second encoder providing module 120 may load the second encoder into the memory. Because complexity of the amino acid sequence of the enzyme and a molecular graph of the compound is very high, the first encoder and the second encoder may first learn a function of the enzyme (e.g., the EC number) and the structural feature of the compound. Here, the first encoder may be an enzyme function learning model and may be referred to as a protein encoder, and the second encoder may be a compound structure characteristic learning model and may be referred to as a chemical encoder.

The combination network construction module 130 may construct a combination network that combines the first encoder provided from the first encoder providing module 110 and the second encoder provided from the second encoder providing module 120. In some embodiments, the combination network construction module 130 may use transfer learning to combine the first encoder and the second encoder. The transfer learning may be a technique that utilizes a weight of an already learned model in an initialization stage of a new model, and may construct the new model by combining the first encoder and the second encoder independently learned. For example, the transfer learning may be implemented to combine outputs of the first encoder and the second encoder and perform a final prediction through an additional prediction layer. Accordingly, the transfer learning may be used to combine the enzyme function learning model with the compound structure characteristic learning model so that whether the enzyme reacts with the compound is learned. The transfer learning may be used to combine the enzyme function learning model with the compound structural feature learning model so that a method in which the enzyme reacts with the compound is learned is adopted. Thus, after each encoder learns a characteristic of a specific data type, combining the learned characteristics may be very useful for learning integrated representations of various data sources. This may enable processing of complex multi-modal data, and may achieve better predictive performance.

In some embodiments, the combination network construction module 130 may combine a first feature extracted from the first encoder and a second feature extracted from the second encoder into one vector through a fully connected layer. That is, an additional prediction layer receiving a vector obtained by combining an output of the first encoder and an output of the second encoder as an input may be defined as the fully connected layer. A feature vector combining the output of the first encoder and the output of the second encoder may be input to the fully connected layer to be processed as integrated information required for the final prediction. Here, the fully connected layer may be a layer in which all nodes of one layer are connected to all nodes of a next layer thereof, and may be used to generate a final output by processing input data. A weight may be assigned to each connection and a bias may be added to each connection so that the weight and the bias are applied when an input value is converted to an output value.

In some embodiments, a combination network (or a coupling network) 23 may apply a nonlinear activation function to add nonlinearity to the fully connected layer to receive a vector combining the first feature extracted from the first encoder and the second feature extracted from the second encoder as an input so that it performs linear transformation by applying the weight and the bias and predicts a reaction probability by nonlinear transformation. In some embodiments, the combination network 23 may apply a sigmoid activation function as the nonlinear activation function. The sigmoid activation function may convert all input values to a value between 0 and 1, and may have an S-shaped curve shape.

The reaction probability prediction module 140 may input an amino acid sequence of the candidate enzyme and graph data for a graph of the target compound to the first encoder and the second encoder, and may perform a calculation predicting a reaction probability in which the candidate enzyme and the target compound will react through the combination network. The reaction probability prediction module 140 may store the calculated reaction probability in a memory.

The learning module 150 may learn the first encoder, the second encoder, and the combination network using a pre-prepared data set.

In some embodiments, the first encoder may predict a characteristic of the enzyme from the amino acid sequence of the enzyme. Here, the characteristic of the enzyme may be classified according to various methods. For example, the characteristic of the enzyme may be classified through gene ontology (GO), protein ontology (PRO), kyto encyclopedia of genes and genomes (KEGG) Brite/Ontology, InterPRO, MetaCyc, cluster of orthologous groups of proteins (COGs), SEED, SCOPe, CATH, or the like. That is, the first encoder may predict the characteristic of the enzyme in a form according to the classification method listed above from the amino acid sequence of the enzyme. Hereinafter, for clarity and convenience of description, embodiments will be mainly described using the EC number as an example. However, a classification method for predicting the characteristic of the enzyme to which an idea of the present disclosure is applied is not limited to a form of the EC number, and may include various types of classification methods listed above.

In some embodiments, the first encoder may learn a reaction feature of the enzyme through a task of predicting the EC number from the amino acid sequence of the enzyme. The EC number may include four levels (e.g., a main level, a sub-level, a sub-sub-level, and a naming level). For example, EC 1.2.1.3 may be the EC number of acetaldehyde dehydrogenase (e.g., $NAD^+$-dependent), and may provide a specific classification as follows.

EC 1: Oxidoreductases - an enzyme that catalyzes an oxidation-reduction reaction

> EC 1.2: an enzyme that uses aldehyde or an oxo group as a donor and uses $NAD^+$ or $NADP^+$ as a receptor
> EC 1.2.1: aldehyde dehydrogenase that uses $NAD^+$ or $NADP^+$ as a receptor
> EC 1.2.1.3: acetaldehyde dehydrogenase

Here, EC 1 may be the main level (or the main layer item), EC 1.2 may be the sub-level (or the sub-layer item), EC 1.2.1 may be the sub-sub-level (or the sub-sub-layer item), and EC 1.2.1.3 may be the naming level (or the naming layer item). Because each level includes multi-classes, a prediction problem of the EC number may correspond to a multi-level multi-class problem.

To solve the multi-level multi-class problem, learning may be performed using Softmax Activation as an output layer. However, a case in which the EC number of the candidate enzyme (i.e., acetaldehyde dehydrogenase) is predicted as 1.2.1.4 or 6.3.5.10 when Softmax Activation is learned may be treated as an incorrect answer. However, from a perspective of the EC number of the multi-level multi-class, the incorrect answer of 1.2.1.4 for the candidate enzyme may be similar to a correct answer, but the incorrect answer of 6.3.5.10 may be completely different from the correct answer, so that a softmax activation learning-based method is not suitable for learning the reaction feature of the enzyme.

In some embodiments, the first encoder may perform multi-class prediction for a multi-level of the EC number using contrastive learning. The first encoder may be learned to generate two positive samples through data augmentation, maximize similarity between the positive samples through a contrastive loss function, and minimize similarity between negative samples. Due to characteristics of data of the EC number, it may be seen that the number of data for each number varies greatly. For example, in Swiss-Prot data of a UniProt database, the number of data of the enzyme corresponding to EC 2.1.3.15 may be 980 but the number of data of the enzyme of EC 1.14.14.138 may be 1 so that there is a data imbalance. The data imbalance may prevent a neural network from learning the reaction feature of the enzyme. To solve the data imbalance problem, the contrastive learning capable of learning similarity and dissimilarity between data may be introduced. In some embodiments, simple framework for contrastive learning of visual representations (SimCLR) may be used as a contrastive learning framework, and a loss function such as Equation 1 below may be used as the contrastive loss function.

(Equation 1)

$$ I_{sim} = -\sum_{i,j} \log \frac{\exp\left(\frac{sim(z_i, z_j)}{\tau}\right)}{\sum_{k=1}^{2N} 1_{[k \neq i]} \exp\left(\frac{sim(z_i, z_j)}{\tau}\right)} $$

In Equation 1 above, $z_i$ and $z_j$ may be an embedding vector of a positive pair, $sim(z_i, z_j)$ may be cosine similarity of $z_i$ and $z_j$, $\tau$ may be a temperature parameter, N may be a batch size, and $1_{[k \neq i]}$ may represent 1 when $k \neq i$.

In some other embodiments, a loss function such as Equation 2 below may be used.

(Equation 2)

$$ I_{sim} = -\sum_{i,j} \log \frac{\mathbb{N}_{i,k} \exp\left(\frac{sim(z_i, z_j)}{\tau}\right)}{\sum_{k=1}^{2N} \mathbb{N}_{i,k} \exp\left(\frac{sim(z_i, z_j)}{\tau}\right)} $$

In Equation 2 above, $\mathbb{N}_{i,k}$ may be a multi-level term, and may have a value of 0 if EC numbers for enzymes (i, k) are the same, may have a value of 0.25 if sub-sub-classes are the same, may have a value of 0.5 if sub-classes are the same, may have a value of 0.75 if main classes are the same, and may have a value of 1 if the main classes are different.

In some embodiments, the second encoder may map the graph of the target compound to a continuous number-based latent space based on a generative model. The generative model may generally include an encoder that maps a material structure that is discrete information into the continuous number-based latent space, and a decoder that reduces a value of the latent space back to the material structure. For example, a generative model based on a variational autoencoder (VAE) may learn the model by reflecting an error with an original structure in the loss function when the material structure is encoded in learning data into the latent space, a small noise is added to the encoded material structure, and then the added small noise is decoded back into the structure. When a material with a similar structure is encoded in a similar position in the latent space and a similar latent space is decoded through the learning process, the similar structure may be generated. By using an encoder portion of the generative model as the second encoder, a latent space value reflecting structural similarity between compounds may be obtained.

In some embodiments, the second encoder may extract a feature for a junction in a unit of a junction tree larger than a unit of an atom. That is, after a material is separated into the junction that is not the unit of the atom and is a meaningful partial structure and a label of the junction, a coupling relationship between junctions, a coupling position, and the like are generated as input data, the latent space may be obtained through the encoder, and the decoder may predict the label, an order, and a coupling position of the junction to be used for decoding of the material. In this case, a loss function such as Equation 3 below may be introduced.

(Equation 3)

$$ Loss = KLLoss(q(z|x), p(z)) + LabelLoss + AdjLoss + AssemLoss + PredLoss $$

In Equation 3 above, $KLLoss(q(z|x), p(z))$ may be a Kullback-Leibler divergence Loss between an encoder transformation value distribution of x and a normal distribution, LabelLoss may be prediction accuracy of the junction, AdjLoss may be prediction accuracy of neighborhood between the junctions, AssemLoss may be prediction accuracy of the coupling position between the junctions, and PredLoss may be a L1 loss of an energy prediction value. In Equation 3 above, the L1 loss may represent a loss function that calculates a difference between a predicted value and an actual value as a sum of absolute errors.

In some embodiments, the second encoder may further include a network that predicts energy of a reactant from a mapping value of the latent space. That is, a neural network that predicts energy from a latent space transformation value of the material may be added to the generative model and a characteristic prediction result may be reflected in the loss function so that the latent space learned by the model is aligned according to a characteristic of the material (i.e., energy). The energy may be used in the alignment of the latent space because a structure and change of the energy are important factors. Through the process, the reactant with a similar structure and energy may have a similar value in the latent space, and an enzyme-reactant reaction prediction model may use a latent space value of the reactant for the learning. Thus, the reactant and the energy may be reflected in the learning.

The final candidate enzyme selection module 160 may select the final candidate enzyme based on the reaction probability calculated by the reaction probability prediction module 140. For example, the final candidate enzyme selection module 160 may select the final candidate enzyme that performs a reaction on the biosynthetic pathway for producing the target compound among candidate enzymes, and may store biosynthetic pathway data that optimizes the biosynthetic pathway using the final candidate enzyme in a memory. In some embodiments, the final candidate enzyme selection module 160 may be implemented to calculate a score for the candidate enzyme and select the final candidate enzyme from the candidate enzymes based on the score.

The present embodiment may search the enzyme capable of reacting with a designated target compound based on an artificial intelligence technology, and may predict a possibility of a reaction with the target compound without being limited to predicting a function of the enzyme through an artificial intelligence model that learns the function of the enzyme and the transfer learning. Accordingly, the embodiments may discover a promising enzyme that may perform a specific reaction well, and may accelerate development of a microbial strain that produces an eco-friendly bio-based material.

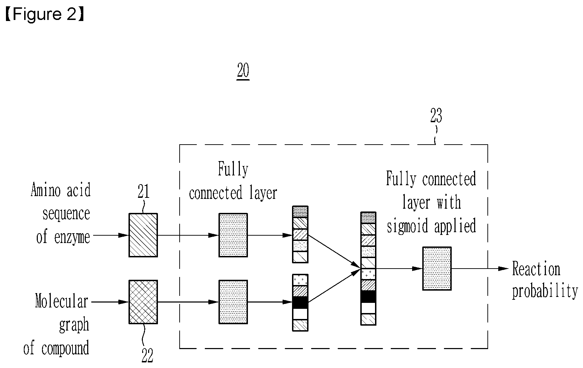

FIG. 2 is a view for describing a device for searching an enzyme according to an embodiment.

Referring to FIG. 2, the device for searching the enzyme according to the embodiment may include a candidate enzyme search model 20. The candidate enzyme search model 20 may include a first encoder 21, a second encoder 22, and the combination network 23. The first encoder 21 may be a protein encoder and may receive an amino acid sequence of the enzyme and may output a first embedding, and the second encoder 22 may be a chemical encoder and may receive a molecular graph of the compound and may output a second embedding. The combination network 23 may combine the first embedding and the second embedding into one vector and may perform a final prediction to output the candidate enzyme and a reaction probability of the target compound.

Descriptions of the embodiments of the present specification may be applied to detailed descriptions of the first encoder 21, the second encoder 22, and the combination network 23.

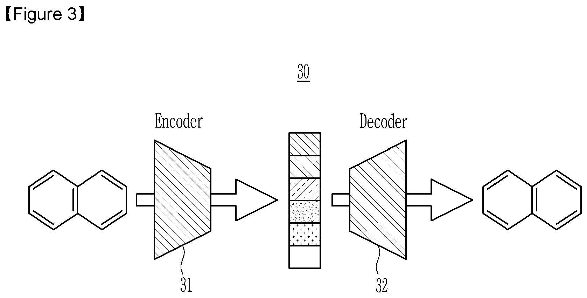

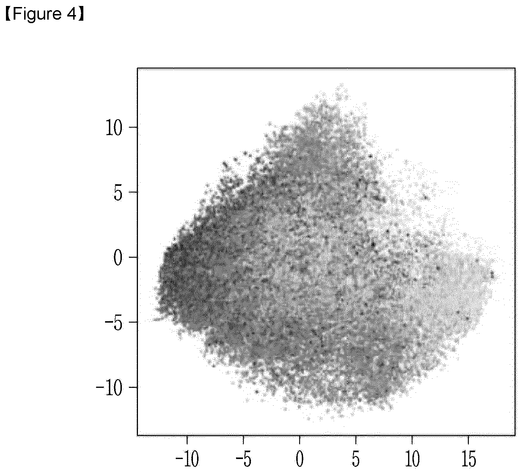

FIG. 3 is a view for describing a generative model related to the second encoder according to an embodiment, and FIG. 4 is a view for describing the second encoder according to an embodiment.

Referring to FIG. 3, a generative model 30 related to the second encoder may include an encoder 31 and a decoder 32, and a latent space value that is an output of the encoder may be used to learn an enzyme-reactant prediction model.

Referring to FIG. 4, a distribution of the latent space and an energy calculation value are shown. A graph of FIG. 4 may show first and second components of principal component analysis (PCA) of the latent space value after the latent space value of a learning material is calculated with the generative model in which the learning is completed. A color of each of points may be the calculated energy value, and it may be seen that the points are well aligned from the left corresponding to a dark color and high energy to the right corresponding to a light color and low energy.

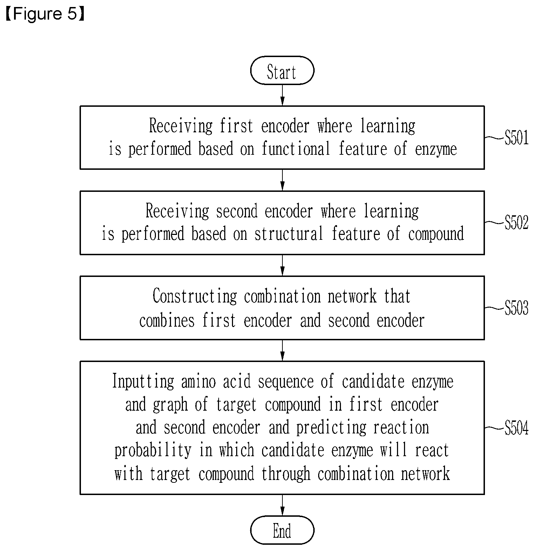

FIG. 5 is a view for describing a method for searching the enzyme according to an embodiment.

Referring to FIG. 5, the method for searching the enzyme according to the embodiment may include a step S501 of receiving the first encoder where learning is performed based on a functional feature of the enzyme, a step S502 of receiving the second encoder where learning is performed based on a structural feature of the compound, a step S503 of constructing the combination network that combines the first encoder with the second encoder, and a step S504 of inputting an amino acid sequence of the candidate enzyme and a graph of the target compound in the first encoder and the second encoder and predicting a reaction probability in which the candidate enzyme will react with the target compound through the combination network.

The method may be implemented to be performed by the computing device including the processor and the memory. In this case, in the step S501, the processor may load the first encoder in which the learning is performed based on the functional feature of the enzyme into the memory, and in the step S502, the processor may load the second encoder in which the learning is performed based on the structural feature of the compound into the memory. In the step S503, the processor may build the combination network that combines the first encoder and the second encoder through the transfer learning, and in the step S504, the processor may input the amino acid sequence of the candidate enzyme and graph data of the target compound in the first encoder and the second encoder, and may calculate the reaction probability in which the candidate enzyme will react with the target compound through the combination network to store the calculated reaction probability in the memory. In the step S505, the processor may select a final candidate enzyme that performs a reaction on the biosynthetic pathway for producing the target compound among the candidate enzymes based on the calculated reaction probability, and may store biosynthetic pathway data obtained by optimizing the biosynthetic pathway using the final candidate enzyme in the memory.

Because descriptions of the embodiments of the present specification are applied to more detailed descriptions of the method, a redundant description thereof will be omitted.

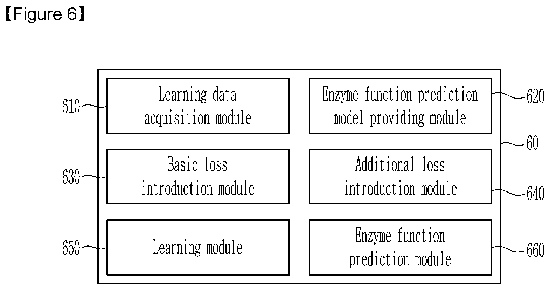

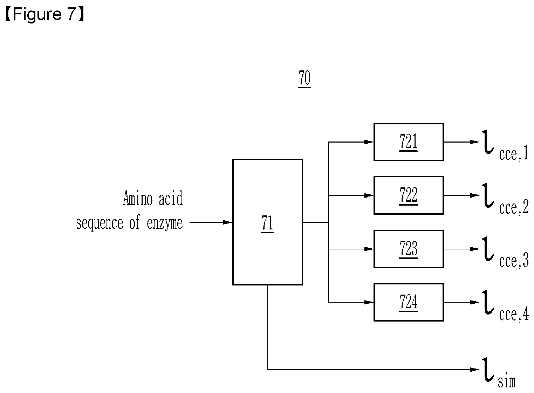

FIG. 6 is a view for describing a device for predicting the function of the enzyme according to an embodiment, and FIG. 7 is a view for describing a device for predicting the function of the enzyme according to an embodiment.

As describeed above, if the learning is performed using Softmax Activation as the output layer to solve the multi-level multi-class problem, the incorrect answer that is similar to the correct answer and the incorrect answer that is completely different from the correct answer may not be treated separately from a perspective of the EC number of the multi-level multi-class. Therefore, the softmax activation learning-based method may not be suitable for learning the reaction feature of the enzyme. To overcome the limitation, improve prediction accuracy for the functional annotation having the hierarchical structure, and solve the data imbalance problem, a total loss function may be defined by introducing a plurality of basic losses corresponding to the hierarchical structure of the functional annotation and an additional loss related to the data augmentation. As a result, an enzyme function prediction model may be learned so that the hierarchical structure of the functional annotation is reflected to position features of enzymes with the same class at each level sufficiently close to each other.

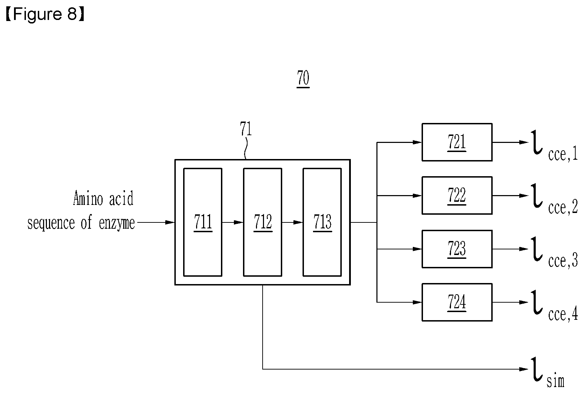

Referring to FIG. 6 and FIG. 7 together, a device for predicting a function of the enzyme 60 may include a learning data acquisition module 610, an enzyme function prediction model providing module 620, a basic loss introduction module 630, an additional loss introduction module 640, a learning module 650, and an enzyme function prediction module 660. The device for predicting the function of the enzyme 60 of FIG. 6 may correspond to a device for predicting a function of the enzyme 70 of each of FIG. 7 and FIG. 8. The first encoder described above with respect to FIGS. 1 to 5 may include an enzyme function prediction model that predicts the function of the enzyme through prediction of functional annotation of a protein. Thus, the enzyme function prediction model included in the first encoder may be learned by the device for predicting the function of the enzyme 60 and the device for predicting the function of the enzyme 70. The first encoder may perform feature extraction based on the functional feature of the enzyme using the enzyme function prediction model.

The learning data acquisition module 610 may acquire learning data related to the functional feature of the enzyme. In some embodiments, the learning data acquisition module 610 may acquire data including information on the amino acid sequence of the enzyme as the learning data.

The enzyme function prediction model providing module 620 may provide an enzyme function prediction model 71. In addition, the enzyme function prediction model providing module 620 may provide a plurality of activation layers 721, 722, 723, and 724 corresponding to a plurality of levels following the enzyme function prediction model 71.

The basic loss introduction module 630 may introduce a plurality of basic losses for each of the plurality of activation layers 721, 722, 723, and 724. The plurality of basic losses may include a first basic loss to an Mth basic loss (where M is an integer greater than or equal to 2). In FIG. 7, the plurality of basic losses including a first basic loss $l_{cce,1}$, a second basic loss $l_{cce,2}$, a third basic loss $l_{cce,3}$, and a fourth basic loss $l_{cce,4}$ are illustrated.

As described above, the functional annotation may include first to Mth layer items (where M is an integer greater than or equal to 2) corresponding to the plurality of levels. In this case, the first basic loss to the Mth basic loss may correspond to the first layer item to the Mth layer item, respectively. For example, if the functional annotation includes the EC number, the functional annotation may include a main layer item, a sub-layer item, a sub-sub-layer item, and a naming layer item. In this case, the main layer item may correspond to the first basic loss $l_{cce,1}$ among the plurality of basic losses, the sub-layer item may correspond to the second basic loss $l_{cce,2}$ among the plurality of basic losses, the sub-sub-layer item may correspond to the third basic loss $l_{cce,3}$ among the plurality of basic losses, and the naming layer item may correspond to the fourth basic loss $l_{cce,4}$ among the plurality of basic losses.

The additional loss introduction module 640 may apply data augmentation to the enzyme function prediction model 71, and may introduce the additional loss. In this case, the enzyme function prediction model 71 may perform multi-class prediction for a multi-level of the functional annotation using the contrastive learning. Due to characteristics of data of the EC number, it may be seen that the number of data for each number varies greatly. For example, in Swiss-Prot data of a UniProt database, the number of data of the enzyme corresponding to EC 2.1.3.15 may be 980 but the number of data of the enzyme of EC 1.14.14.138 may be 1 so that there is a data imbalance. The data imbalance may prevent the neural network from learning the reaction feature of the enzyme. To solve the data imbalance problem, the contrastive learning capable of learning similarity and dissimilarity between data may be introduced. For example, the additional loss introduction module 640 may introduce the additional loss to generate positive samples through the data augmentation, maximize similarity between the positive samples, and minimize similarity between negative samples.

The learning module 650 may define a total loss function based on the plurality of basic losses introduced by the basic loss introduction module 630 and the additional loss introduced by the additional loss introduction module 640. In some embodiments, the learning module 650 may define a total loss function $l$ according to Equation 4 below.

## (Equation 4)

$$I = \sum_{k=1}^{M} I_{cce,k} + I_{sim}$$

In Equation 4 above, $I_{cce,k}$ may be a kth basic loss among the plurality of basic losses (where k is an integer greater than or equal to 1 and less than or equal to M), and $I_{sim}$ may be the additional loss.

In some embodiments, the additional loss introduction module 640 may define the additional loss according to Equation 5 below.

## (Equation 5)

$$I_{sim} = -\sum_{i,j} \log \frac{\exp\left(\frac{sim(z_i, z_j)}{T}\right)}{\sum_{k=1}^{2N} 1_{[k \neq i]} \exp\left(\frac{sim(z_i, z_j)}{T}\right)}$$

In Equation 5 above, $z_i$ and $z_j$ may be an embedding vector of a positive pair, $sim(z_i, z_j)$ may be cosine similarity of $z_i$ and $z_j$, $\tau$ may be a temperature parameter, N may be a batch size, and $1_{[k \neq i]}$ may represent 1 when $k \neq i$.

In some embodiments, the additional loss introduction module 640 may define the additional loss according to Equation 6 below.

## (Equation 6)

$$I_{sim} = -\sum_{i,j} \log \frac{\mathbb{N}_{i,k} \exp\left(\frac{sim(z_i, z_j)}{T}\right)}{\sum_{k=1}^{2N} \mathbb{N}_{i,k} \exp\left(\frac{sim(z_i, z_j)}{T}\right)}$$

In Equation 6 above, $\mathbb{N}_{i,k}$ may be a multi-level term, $z_i$ and $z_j$ may be an embedding vector of a positive pair, $sim(z_i, z_j)$ may be cosine similarity of $z_i$ and $z_j$, $\tau$ may be a temperature parameter, N may be a batch size, and $1_{[k \neq i]}$ may represent 1 when $k \neq i$.

In some embodiments, the learning module 650 may define a total loss function I according to Equation 7 below. A difference between Equation 7 and Equation 4 is that a predetermined weight is applied to each of the basic loss and the additional loss.

## (Equation 7)

$$I = \sum_{k=1}^{M} w_{cce,k} \cdot I_{cce,k} + w_{sim} \cdot I_{sim}$$

In Equation 7 above, $I_{cce,k}$ may be a kth basic loss among the plurality of basic losses (where k is an integer greater than or equal to 1 and less than or equal to M), $I_{sim}$ may be the additional loss, $w_{cce,k}$ may be a predetermined weight for the kth basic loss, and $w_{sim}$ may be a predetermined weight for the additional loss.

If the functional annotation includes the EC number, $\mathbb{N}_{i,k}$ may be set to have a value of 0 if EC numbers for enzymes (i, k) are the same, may be set to have a value of 0.25 if sub-sub-classes are the same, may be set to have a value of 0.5 if sub-classes are the same, may be set to have a value of 0.75 if main classes are the same, and may be set to have a value of 1 if the main classes are different. The hierarchical structure of the functional annotation may be reflected by the setting to position features of enzymes with the same class at each level sufficiently close to each other. Thus, the prediction accuracy for the functional annotation having the hierarchical structure may be improved, and the data imbalance problem may be solved.

Additionally, the learning module 650 may learn the enzyme function prediction model 71 based on the learning data acquired by the learning data acquisition module 610 and the total loss function. For example, the learning module 650 may change a parameter value of the enzyme function prediction model 71 based on the learning data and the total loss function.

The enzyme function prediction module 660 may predict the functional annotation using the enzyme function prediction model 71 in which the learning is completed. In some embodiments, as described above with respect to FIGS. 1 to 5, a result predicted by the enzyme function prediction module 660 based on the functional feature of the enzyme may be combined with another result predicted based on the structural feature of the compound to be used to predict a possibility of a reaction between the candidate enzyme and the target compound.

The present embodiment may define the total loss function by introducing the plurality of basic losses corresponding to the hierarchical structure and the additional loss related to the data augmentation to perform the learning of the enzyme function prediction model. Thus, the embodiment may reflect the hierarchical structure of the functional annotation to position features of enzymes with the same class at each level sufficiently close to each other so that it improves the prediction accuracy for the functional annotation having the hierarchical structure and solves the data imbalance problem. In addition, the present embodiment may search the candidate enzyme capable of reacting with a designated target compound based on the artificial intelligence technology, and may be applied to predict the possibility of the reaction with the target compound without being limited to predicting the function of the enzyme through the artificial intelligence model that learns the function of the enzyme and the transfer learning so that it discovers a promising enzyme that may perform the specific reaction well and accelerates development of a microbial strain that produces the eco-friendly bio-based material.

FIG. 8 is a view for describing the device for predicting the function of the enzyme according to an embodiment.

Referring to FIG. 8, in the device for predicting the function of the enzyme 70 according to the embodiment, the enzyme function prediction model 71 may include an embedding layer 711, an attention layer 712, and a feed forward network layer 713. As described in FIG. 7, the plurality of activation layers 721, 722, 723, and 724 may be formed following the enzyme function prediction model 71.

The embedding layer 711 may convert input data (e.g., data including information on the amino acid sequence of the enzyme) into a high-dimensional vector. That is, the embedding layer may digitize the input data to allow the model to process the digitized input data.

The attention layer 712 may receive a vector embedded by the embedding layer 711, and may serve to emphasize an important portion by assigning a weight to each element of an input sequence. In some embodiments, the attention layer 712 may include a fast attention via orthogonal random feature (FAVOR+) attention layer. As the FAVOR+ attention layer is adopted, a computational efficiency may be increased by accelerating attention calculation using an orthogonal random feature.

The feed forward network layer 713 may process an output of the attention layer 712 to apply a nonlinear transformation, and may independently transform a feature of each position. For example, the feed forward network layer 713 may generate an output vector by applying two linear transformations (e.g., the fully connected layer) and a nonlinear activation function (e.g., ReLU) between them.

According to the present embodiment, the embedding layer 711, the attention layer 712, and the feed forward network layer 713 may complement each other to learn a complex pattern and an interaction of amino acid sequence data of the enzyme, and high performance, efficient computation, and a powerful expression capability may be expected in prediction of functional annotation.

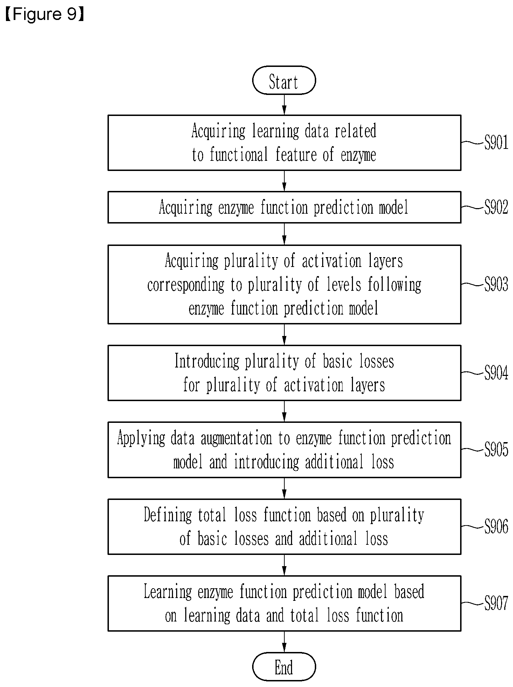

FIG. 9 is a view for describing a method for searching the enzyme based on prediction of the function of the enzyme according to an embodiment.

Referring to FIG. 9, the method for searching the enzyme according to the embodiment may include a step S901 of acquiring learning data related to the functional feature of the enzyme, a step S902 of acquiring the enzyme function prediction model, a step S903 of acquiring the plurality of activation layers corresponding to the plurality of levels following the enzyme function prediction model, a step S904 of respectively introducing the plurality of basic losses for the plurality of activation layers, a step S905 of applying the data augmentation to the enzyme function prediction model and introducing the additional loss, a step S906 of defining the total loss function based on the plurality of basic losses and the additional loss, and a step S907 of learning the enzyme function prediction model based on the learning data and the total loss function. The method may be implemented to be performed by the computing device including the processor and the memory. In this case, each step S901-S907 may be performed by the processor.

Because descriptions of the embodiments of the present specification are applied to more detailed descriptions of the method, a redundant description thereof will be omitted.

FIG. 10 is a view for describing the computing device according to an embodiment.

Referring to FIG. 10, the method and the device for searching the enzyme according to the embodiments may be implemented using the computing device 50. The computing device 50 may be implemented as various types of electronic devices, servers, or devices similar thereto, and their functions may be implemented through a combination of software and hardware.

The computing device 50 may include at least one of the processor 510, a memory 530, a user interface input device 540, a user interface output device 550, and a storage device 560 that communicate through a bus 520. The computing device 50 may also include a network interface 570 electrically connected to a network 40. The network interface 570 may transmit or receive a signal to or from another entity through the network 40.

The processor 510 may be implemented as various types of calculation devices such as a micro controller unit (MCU), an application processor (AP), a central processing unit (CPU), a graphic processing unit (GPU), a neural processing unit (NPU), and a quantum processing unit (QPU). The processor 510 may be a semiconductor device that executes a command stored in the memory 530 or the storage device 560, and may perform a key role of a system. A program code and data stored in the memory 530 or the storage device 560 may instruct the processor 510 to perform a specific task so that they enable an overall operation of the system. The processor 510 may be configured to implement various functions and methods described above with respect to FIGS. 1 to 9.

The memory 530 and the storage device 560 may include various forms of volatile or non-volatile storage media for storing and accessing data of the system. For example, the memory 530 may include a read-only memory (ROM) 531 and a random access memory (RAM) 532. In some embodiments, the memory 530 may be embedded inside the processor 510, and in this case, a data transmission speed between the memory 530 and the processor 510 may be very fast. In some other embodiments, the memory 530 may be disposed outside the processor 510, and in this case, the memory 530 may be connected to the processor 510 via various data buses or interfaces. This connection may be made through a variety of known means such as a Peripheral Component Interconnect Express (PCIe) interface for high-speed data transmission or a memory controller.

In some embodiments, at least some components or functions of the method and the device for searching the enzyme according to the embodiments may be implemented as a program or software executed in the computing device 50, and the program or the software may be stored in a computer-readable recording medium or storage medium. For example, the computer-readable recording medium or storage medium according to an embodiment may be a medium in which a program for executing steps included in implementation of the method and the device for searching the enzyme according to the embodiments is recorded in a computer including the processor 510 executing a program or a command stored in the memory 530 or the storage device 560.

In some embodiments, at least some components or functions of the method and the device for searching the enzyme according to the embodiments may be implemented using hardware or a circuit of the computing device 50, or may be implemented as separate hardware or a separate circuit that may be electrically connected to the computing device 50.

In some embodiments, one or more non-transitory computer-readable media including an instruction capable of being executed by the computing device 50 may be provided, and the instruction may allow the computing device 50 to perform an operation when it is executed by one or more processors of the computing device 50. The operation may include an element, a function, a step, or the like described in the present specification with respect to the method and the device for searching the enzyme according to the embodiments.

According to the embodiments, the artificial intelligence technology based on the functional feature of the enzyme and the structural feature of the compound may be used to search the enzyme capable of reacting with the designated target compound. According to the embodiments, through the artificial intelligence model that learns the function of the enzyme and the transfer learning, it is possible to predict the possibility of the reaction with the target compound without being limited to predicting the function of the enzyme. Accordingly, according to the embodiments, it is possible to discover the promising enzyme that may perform the specific reaction well and accelerate development of the microbial strain that produces the eco-friendly bio-based material.

Additionally, the embodiments may provide prediction of the function of the enzyme, and in order to perform learning of an enzyme function prediction model, the embodiments may define a total loss function by introducing a plurality of basic losses corresponding to a hierarchical structure and an additional loss related to data augmentation. Accordingly, the embodiments may reflect the hierarchical structure of the functional annotation to position features of enzymes with the same class at each level sufficiently close to each other, and may improve the prediction accuracy for the functional annotation having the hierarchical structure and may solve the data imbalance problem.

While this disclosure has been described in connection with what is presently considered to be practical embodiments, it should be understood that the disclosure is not limited to the disclosed embodiments, but, on the contrary, is intended to cover various modifications and equivalent arrangements included within the spirit and scope of the appended claims.

**Claims**

1. A method for searching an enzyme performed by a computing device including a processor and a memory, comprising:

loading, by the processor, a first encoder in which learning is performed based on a functional feature of the enzyme into the memory;

loading, by the processor, a second encoder in which learning is performed based on a structural feature of a compound into the memory;

constructing, by the processor, a combination network that combines the first encoder and the second encoder through transfer learning;

inputting, by the processor, an amino acid sequence of a candidate enzyme and graph data of a target compound in the first encoder and the second encoder and calculating, by the processor, a reaction probability in which the candidate enzyme and the target compound will react through the combination network to store the calculated reaction probability in the memory; and

selecting, by the processor, a final candidate enzyme that performs a reaction on a biosynthetic pathway for producing the target compound among candidate enzymes based on the calculated reaction probability and storing, by the processor, biosynthetic pathway data that optimizes the biosynthetic pathway using the final candidate enzyme in the memory.

2. The method of claim 1, wherein the constructing of the combination network includes combining, by the processor, a first feature extracted from the first encoder and a second feature extracted from the second encoder into one vector through a fully connected layer, and the calculating the reaction probability to store the calculated reaction probability in the memory includes nonlinearly transforming, by the processor, the vector by applying a nonlinear activation function to the fully connected layer to calculate the reaction probability.

3. The method of claim 1, wherein the first encoder learns a reaction feature of the enzyme through a task of predicting an enzyme commission (EC) number from an amino acid sequence of the enzyme, and performs multi-class prediction for a multi-level of the enzyme commission number using contrastive learning.

4. The method of claim 3, wherein the first encoder is learned to generate two positive samples through data augmentation, maximize similarity between the positive samples through a contrastive loss function, and minimize similarity between negative samples.

5. The method of claim 1, wherein the second encoder maps a graph of the target compound to a continuous number-based latent space based on a generative model, and extracts a feature for a junction in a unit of a junction tree greater than a unit of an atom.

6. The method of claim 5, wherein the second encoder further includes a network that predicts energy of a reactant from a mapping value of the latent space.

7. The method of claim 1, wherein the first encoder includes an enzyme function prediction model that predicts enzyme function through prediction of functional annotation of a protein; and
the method further comprises:

acquiring, by the processor, learning data related to the functional feature of the enzyme;
respectively introducing, by the processor, a plurality of basic losses for a plurality of activation layers corresponding to a plurality of levels;
applying, by the processor, data augmentation to the enzyme function prediction model and introducing, by the processor, an additional loss;
defining, by the processor, a total loss function based on the plurality of basic losses and the additional loss; and
learning, by the processor, the enzyme function prediction model based on the learning data and the total loss function.

8. The method of claim 7, wherein the plurality of basic losses include a first basic loss to an Mth basic loss, wherein M is an integer greater than or equal to 2; and

the defining of the total loss function includes defining the total loss function (I) according to Equation 1 below:

## (Equation 1)

$$I = \sum_{k=1}^{M} w_{cce,k} \cdot I_{cce,k} + w_{sim} \cdot I_{sim},$$

wherein in Equation 1 above, $I_{cce,k}$ is a kth basic loss among the plurality of basic losses (where k is an integer greater than or equal to 1 and less than or equal to M), $I_{sim}$ is the additional loss, $w_{cce,k}$ is a predetermined weight for the kth basic loss, and $w_{sim}$ is a predetermined weight for the additional loss.

9. The method of claim 8, wherein the additional loss is defined according to Equation 2 below:

## (Equation 2)

$$I_{sim} = -\sum_{i,j} \log \frac{\exp\left(\frac{sim(z_i, z_j)}{\tau}\right)}{\sum_{k=1}^{2N} 1_{[k \neq i]} \exp\left(\frac{sim(z_i, z_j)}{\tau}\right)},$$

wherein in Equation 2 above, $z_i$ and $z_j$ are an embedding vector of a positive pair, $sim(z_i, z_j)$ is cosine similarity of $z_i$ and $z_j$, $\tau$ is a temperature parameter, N is a batch size, and $1_{[k \neq i]}$ represents 1 when $k \neq i$.

10. The method of claim 8, wherein the additional loss is defined according to Equation 3 below:

## (Equation 3)

$$I_{sim} = -\sum_{i,j} \log \frac{\mathbb{N}_{i,k} \exp\left(\frac{sim(z_i, z_j)}{\tau}\right)}{\sum_{k=1}^{2N} \mathbb{N}_{i,k} \exp\left(\frac{sim(z_i, z_j)}{\tau}\right)},$$

wherein in Equation 3 above, $\mathbb{N}_{i,k}$ is a multi-level term, $z_i$ and $z_j$ are an embedding vector of a positive pair, $sim(z_i, z_j)$ is cosine similarity of $z_i$ and $z_j$, $\tau$ is a temperature parameter, N is a batch size, and $1_{[k \neq i]}$ represents 1 when k # i.

11. A device for searching an enzyme, comprising:

one or more non-transitory computer-readable media that include an instruction; and
one or more processors that perform an operation by executing the instruction, wherein the operation comprises:

loading a first encoder in which learning is performed based on a functional feature of the enzyme into the media;
loading a second encoder in which learning is performed based on a structural feature of a compound into the media;
constructing a combination network that combines the first encoder and the second encoder through transfer learning;
inputting an amino acid sequence of a candidate enzyme and graph data of a target compound in the first encoder and the second encoder and calculating a reaction probability in which the candidate enzyme and the target compound will react through the combination network to store the calculated reaction probability in the media; and
selecting a final candidate enzyme that performs a reaction on a biosynthetic pathway for producing the target compound among candidate enzymes based on the calculated reaction probability and storing biosynthetic pathway data that optimizes the biosynthetic pathway using the final candidate enzyme in the media.

12. The device of claim 11, wherein the constructing of the combination network includes combining a first feature extracted from the first encoder and a second feature extracted from the second encoder into one vector through a fully connected layer, and the calculating of the reaction probability includes nonlinearly transforming the vector by applying a nonlinear activation function to the fully connected layer to predict the reaction probability.

13. The device of claim 11, wherein the first encoder learns a reaction feature of the enzyme through a task of predicting an enzyme commission (EC) number from an amino acid sequence of the enzyme, and performs multi-class prediction for a multi-level of the enzyme commission number using contrastive learning.

14. The device of claim 13, wherein the first encoder is learned to generate two positive samples through data augmentation, maximize similarity between the positive samples through a contrastive loss function, and minimize similarity between negative samples.

15. The device of claim 11, wherein the second encoder maps a graph of the target compound to a continuous number-based latent space based on a generative model, and extracts a feature for a junction in a unit of a junction tree greater than a unit of an atom.

16. The device of claim 15, wherein the second encoder further includes a network that predicts energy of a reactant from a mapping value of the latent space.

17. The device of claim 11, wherein the first encoder includes an enzyme function prediction model that predicts enzyme function through prediction of functional annotation of a protein; and
the operation further comprises:

acquiring learning data related to the functional feature of the enzyme;
respectively introducing a plurality of basic losses for a plurality of activation layers corresponding to a plurality of levels;
applying data augmentation to the enzyme function prediction model and introducing an additional loss;
defining a total loss function based on the plurality of basic losses and the additional loss; and
learning the enzyme function prediction model based on the learning data and the total loss function.

18. The device of claim 17, wherein the plurality of basic losses include a first basic loss to an Mth basic loss, wherein M is an integer greater than or equal to 2; and

the defining of the total loss function includes defining the total loss function (l) according to Equation 1 below:

(Equation 1)

$$l = \sum_{k=1}^{M} w_{cce,k} \cdot l_{cce,k} + w_{sim} \cdot l_{sim},$$

wherein in Equation 1 above, $l_{cce,k}$ is a kth basic loss among the plurality of basic losses (where k is an integer greater than or equal to 1 and less than or equal to M), $l_{sim}$ is the additional loss, $w_{cce,k}$ is a predetermined weight for the kth basic loss, and $w_{sim}$ is a predetermined weight for the additional loss.

19. The device of claim 18, wherein the additional loss is defined according to Equation 2 below:

(Equation 2)

$$l_{sim} = -\sum_{i,j} \log \frac{\exp\left(\frac{sim(z_i, z_j)}{\tau}\right)}{\sum_{k=1}^{2N} 1_{[k \neq i]} \exp\left(\frac{sim(z_i, z_j)}{\tau}\right)},$$

wherein in Equation 2 above, $z_i$ and $z_j$ are an embedding vector of a positive pair, $sim(z_i, z_j)$ is cosine similarity of $z_i$ and $z_j$, $\tau$ is a temperature parameter, N is a batch size, and $1_{[k \neq i]}$ represents 1 when $k \neq i$.

20. The device of claim 18, wherein the additional loss is defined according to Equation 3 below:

(Equation 3)

$$l_{sim} = -\sum_{i,j} \log \frac{\mathbb{N}_{i,k} \exp\left(\frac{sim(z_i, z_j)}{\tau}\right)}{\sum_{k=1}^{2N} \mathbb{N}_{i,k} \exp\left(\frac{sim(z_i, z_j)}{\tau}\right)},$$

wherein in Equation 3 above, $\mathbb{N}_{i,k}$ is a multi-level term, $z_i$ and $z_j$ are an embedding vector of a positive pair, $sim(z_i, z_j)$ is cosine similarity of $z_i$ and $z_j$, $\tau$ is a temperature parameter, N is a batch size, and $1_{[k \neq i]}$ represents 1 when $k \neq i$.

【Figure 1】

| | |
|---|---|
| First encoder providing module | Second encoder providing module |
| Combination network construction module | Reaction probability prediction module |
| Learning module | Final candidate enzyme selection module |

【Figure 2】

20

23

Amino acid
sequence
of enzyme

21

Molecular
graph
of compound

22

Fully
connected layer

Fully connected
layer with
sigmoid applied

Reaction
probability

【Figure 3】

【Figure 4】

【Figure 5】

```
                    ( Start )
                       │
                       ▼
┌─────────────────────────────────────────────┐
│         Receiving first encoder where learning│ ~S501
│     is performed based on functional feature of enzyme│
└─────────────────────────────────────────────┘
                       │
                       ▼
┌─────────────────────────────────────────────┐
│        Receiving second encoder where learning│ ~S502
│    is performed based on structural feature of compound│
└─────────────────────────────────────────────┘
                       │
                       ▼
┌─────────────────────────────────────────────┐
│          Constructing combination network that│ ~S503
│         combines first encoder and second encoder│
└─────────────────────────────────────────────┘
                       │
                       ▼
┌─────────────────────────────────────────────┐
│   Inputting amino acid sequence of candidate enzyme│
│      and graph of target compound in first encoder│
│        and second encoder and predicting reaction│ ~S504
│     probability in which candidate enzyme will react│
│    with target compound through combination network│
└─────────────────────────────────────────────┘
                       │
                       ▼
                    ( End )
```

【Figure 6】

| | |
|---|---|
| 610 — Learning data acquisition module | Enzyme function prediction model providing module — 620 |
| 630 — Basic loss introduction module | Additional loss introduction module — 640 |
| 650 — Learning module | Enzyme function prediction module — 660 |

60

【Figure 7】

$$\underline{70}$$

Amino acid sequence of enzyme → $\underline{71}$

$\underline{721}$ → $l_{cce,1}$

$\underline{722}$ → $l_{cce,2}$

$\underline{723}$ → $l_{cce,3}$

$\underline{724}$ → $l_{cce,4}$

$l_{sim}$

【Figure 8】

【Figure 9】

```
                    ┌──────────┐
                    │   Start  │
                    └────┬─────┘
                         │
         ┌───────────────▼───────────────┐
         │  Acquiring learning data related │──S901
         │   to functional feature of enzyme │
         └───────────────┬───────────────┘
                         │
         ┌───────────────▼───────────────┐
         │ Acquiring enzyme function prediction model │──S902
         └───────────────┬───────────────┘
                         │
         ┌───────────────▼───────────────┐
         │  Acquiring plurality of activation layers │
         │ corresponding to plurality of levels following │──S903
         │     enzyme function prediction model │
         └───────────────┬───────────────┘
                         │
         ┌───────────────▼───────────────┐
         │   Introducing plurality of basic losses │──S904
         │    for plurality of activation layers │
         └───────────────┬───────────────┘
                         │
         ┌───────────────▼───────────────┐
         │ Applying data augmentation to enzyme function prediction │──S905
         │      model and introducing additional loss │
         └───────────────┬───────────────┘
                         │
         ┌───────────────▼───────────────┐
         │  Defining total loss function based on plurality │──S906
         │    of basic losses and additional loss │
         └───────────────┬───────────────┘
                         │
         ┌───────────────▼───────────────┐
         │ Learning enzyme function prediction model based │──S907
         │   on learning data and total loss function │
         └───────────────┬───────────────┘
                         │
                    ┌────▼─────┐
                    │    End   │
                    └──────────┘
```

【Figure 10】

## INTERNATIONAL SEARCH REPORT

| International application No. |
| --- |
| **PCT/KR2025/010584** |

| A. | CLASSIFICATION OF SUBJECT MATTER |
| --- | --- |

**G16B 40/00**(2019.01)i; **G16B 15/20**(2019.01)i; **G16B 20/00**(2019.01)i; **G06N 3/08**(2006.01)i; **C12Q 1/00**(2006.01)i; **G16B 40/20**(2019.01)i; **G16C 20/70**(2019.01)i; **G16B 30/10**(2019.01)i; **G16C 20/30**(2019.01)i; **G16C 20/40**(2019.01)i

According to International Patent Classification (IPC) or to both national classification and IPC

| B. | FIELDS SEARCHED |
| --- | --- |

Minimum documentation searched (classification system followed by classification symbols)

G16B 40/00(2019.01); G16B 15/30(2019.01); G16B 30/00(2019.01); G16C 20/10(2019.01); G16C 20/20(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (MOIP internal) & keywords: 인코더(encoder), 효소(enzyme), 서열(sequence), 화합물(compound), 구조(structure), 그래프(graph), 반응 확률(reaction probability), 생합성 경로(biosynthetic pathway)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | WEI, Bomin et al. DeepLPI: a novel deep learning-based model for protein-ligand interaction prediction for drug repurposing. Scientific Reports. 28 October 2022 (online publication date), vol. 12, article no. 18200, pp. 1-11. <br> see pages 2-3, 9; figure 2. | 1-20 |
| A | KROLL, Alexander et al. A general model to predict small molecule substrates of enzymes based on machine and deep learning. Nature Communications. 15 May 2023 (online publication date), vol. 14, article no. 2787, pp. 1-13. <br> see abstract; pages 9-10. | 1-20 |
| A | CN 117409872 A (ZHONGXIN FUTURE (BEIJING) TECHNOLOGY CO., LTD.) 16 January 2024 (2024-01-16) <br> see claims 1, 4. | 1-20 |
| A | CN 116417062 A (OCEAN UNIVERSITY OF CHINA) 11 July 2023 (2023-07-11) <br> see entire document. | 1-20 |

☑ Further documents are listed in the continuation of Box C.     ☑ See patent family annex.

| | |
| --- | --- |
| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
| --- | --- |
| **17 October 2025** | **18 October 2025** |

| Name and mailing address of the ISA/KR | Authorized officer |
| --- | --- |
| **Korean Intellectual Property Office** <br> **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2025/010584** |

| C. | DOCUMENTS CONSIDERED TO BE RELEVANT | |
| --- | --- | --- |
| Category* | Citation of document, with indication, where appropriate, of the relevant passages | Relevant to claim No. |
| A | CHEN, Xiying et al. DEAttentionDTA: protein-ligand binding affinity prediction based on dynamic embedding and self-attention. Bioinformatics. 24 May 2024, vol. 40, no. 6, btae319, pp. 1-10.<br>see entire document. | 1-20 |

Form PCT/ISA/210 (second sheet) (July 2022)

**INTERNATIONAL SEARCH REPORT**
Information on patent family members

International application No.

**PCT/KR2025/010584**

| Patent document cited in search report | | | Publication date (day/month/year) | Patent family member(s) | | | Publication date (day/month/year) |
|---|---|---|---|---|---|---|---|
| CN | 117409872 | A | 16 January 2024 | None | | | |
| CN | 116417062 | A | 11 July 2023 | CN | 116417062 | B | 15 September 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020240095089
- KR 1020240100992